# EUROPEAN PATENT APPLICATION

(11) **EP 4 052 740 A2**
(43) Date of publication of application: **07.09.2022**
(21) Application number: 20880587.9
(22) Date of filing: 29.10.2020
(51) Int. Cl.: A61M 5/168, A61M 5/165, A61M 5/36, A61M 5/14

(54) **LIQUID MEDICINE FLOW RATE REGULATING DEVICE**

(30) Priority: 01.11.2019 KR 20190138896; 06.04.2020 KR 20200041584
(71) Applicant: Kim, Yong Hyun, Goyang-si, Gyeonggi-do 10483 (KR)
(72) Inventor: Kim, Yong Hyun, Goyang-si, Gyeonggi-do 10483 (KR)
(74) Representative: Santarelli
(86) International application number: PCT/KR2020/014901
(87) International publication number: WO 2021/086048

(57) **Abstract**

A medicinal liquid flow control apparatus includes a medicinal liquid flow path that guides a flow of a medicinal liquid. The medicinal liquid flow path includes an inlet flow path through which the medicinal liquid is introduced, a plurality of branch flow paths branching from the inlet flow path, and an outlet flow path to which the plurality of branch flow paths join and through which the medicinal liquid flows out. The medicinal liquid flow control apparatus includes a plurality of transfer tubes configured to correspond to the plurality of branch flow paths, respectively, each of the transfer tubes forming a capillary flow path constituting at least a portion of a corresponding one of the plurality of branch flow paths, and a valve configured to control a flow rate of the medicinal liquid flowing out through the outlet flow path by selectively opening and closing the plurality of branch flow paths.

## Description

### TECHNICAL FIELD

The present disclosure relates to a medicinal liquid flow control apparatus.

### BACKGROUND

There is known a medicinal liquid injection apparatus for injecting a medicinal liquid (e.g., an injection solution) in a liquid state into a patient in order to supply the medicinal liquid to the patient. By using such a medicinal liquid injection apparatus, the medicinal liquid in a predetermined storage space passes through a passage (e.g., the internal spaces of the medicinal liquid injection tube and the injection needle) connected to the patient, and is introduced into the body of the patient.

For medical purposes, there is known an apparatus including a medicinal liquid transfer tube that forms a capillary flow path to prevent a medicinal liquid from being injected into a patient's body too quickly and to slowly inject the medicinal liquid into the body for a relatively long time. By allowing the medicinal liquid to pass through the medicinal liquid transfer tube, the flow rate of the medicinal liquid flowing along the passage of this medicinal liquid injection apparatus is reduced.

### SUMMARY

A conventional medicinal liquid injection apparatus provided with a capillary flow path is configured to reduce the flow rate of a medicinal liquid, but is not configured to control the flow rate of a medicinal liquid administered to a patient. Therefore, it is difficult to control the flow rate of the medicinal liquid administered to the patient based on a patient's condition or type of disease.

Embodiments of the present disclosure improve on or solve at least some problems of the conventional medicinal liquid injection apparatus. To this end, a plurality of embodiments provide a medicinal liquid flow control apparatus configured to control the flow rate of a medicinal liquid when injecting the medicinal liquid into a body.

Embodiments according to an aspect of the present disclosure relate to a medicinal liquid flow control apparatus. In a medicinal liquid flow control apparatus including a medicinal liquid flow path that guides a flow of a medicinal liquid, the medicinal liquid flow path includes an inlet flow path through which the medicinal liquid is introduced, a plurality of branch flow paths branching from the inlet flow path, and an outlet flow path to which the plurality of branch flow paths join and through which the medicinal liquid flows out. The medicinal liquid flow control apparatus according to a representative embodiment includes: a plurality of transfer tubes configured to correspond to the plurality of branch flow paths, respectively, each of the transfer tubes forming a capillary flow path constituting at least a portion of a corresponding one of the plurality of branch flow paths; and a valve configured to control a flow rate of the medicinal liquid flowing out through the outlet flow path by selectively opening and closing the plurality of branch flow paths.

In an embodiment, the plurality of transfer tubes include at least two transfer tubes configured to allow the medicinal liquid to flow at different flow rates.

In an embodiment, the medicinal liquid flow control apparatus may further include a plurality of pressing members configured to correspond to the plurality of branch flow paths, respectively, and configured to be individually movable. The valve may be configured to selectively open and close the plurality of branch flow paths by being pressed or released in accordance with a movement of the pressing member.

In an embodiment, the medicinal liquid flow control apparatus may further include an actuator configured to move the pressing members.

In an embodiment, the actuator may be provided with a plurality of recesses to be spaced apart from each other such that the plurality of pressing members are accommodated in the plurality of recesses, respectively. The valve may be configured to be elastically restored and press the pressing members in a recessed direction of the recesses.

In an embodiment, the actuator may be configured to be actuated in a state in which the recesses move in a direction transverse to the recessed direction thereof. The plurality of pressing members may be selectively received in the plurality of recesses when the actuator is actuated.

In an embodiment, the valve may be configured to open a corresponding branch flow path in a state in which the pressing members are accommodated in the recesses.

In an embodiment, the medicinal liquid flow control apparatus may further include a guide member disposed between the valve and the plurality of pressing members to guide the movement of the plurality of pressing members.

In an embodiment, the medicinal liquid flow control apparatus may further include: a plurality of pressing members configured to correspond to the plurality of branch flow paths, respectively, and configured to be individually movable; an actuator configured to move the pressing members; and an operation member removably engaged with the actuator and configured to actuate the actuator in a state of being engaged with the actuator.

In an embodiment, the operation member may be provided with an engaging portion, and the actuator may be formed with a counterpart engaging portion in which the engaging portion is accommodated.

In an embodiment, the valve may be configured to be elastically deformed by being pressed and elastically restored by being released.

In an embodiment, the medicinal liquid flow control apparatus may further include a valve housing provided with a plurality of valve spaces covered by the valve, each of the valve spaces constituting a portion of a corresponding one of the plurality of branch flow paths.

In an embodiment, the valve housing may be provided with a plurality of valve holes that allow the plurality of valve spaces and the capillary flow paths of the plurality of transfer tubes to respectively communicate with each other.

In an embodiment, the medicinal liquid flow control apparatus may further include a plurality of pressing members configured to correspond to the plurality of branch flow paths, respectively, and configured to be individually movable. The valve may be configured to close the valve holes when pressed by the pressing members.

In an embodiment, one of the plurality of valve spaces may be provided with a connection hole constituting a portion of the outlet flow path.

In an embodiment, the medicinal liquid flow control apparatus may further include a communication flow path configured to communicate two valve spaces adjacent to each other among the plurality of valve spaces.

In an embodiment, one of the plurality of valve spaces may be provided with a connection hole constituting a portion of the outlet flow path. In a state in which the valve opens the valve holes and in a state in which the valve closes the valve holes, the connection hole and the communication flow path may be configured to maintain an opened state.

In an embodiment, the valve may include a plurality of protrusions that protrude toward the plurality of valve spaces and are configured to close the corresponding branch flow paths, respectively.

In an embodiment, the medicinal liquid flow control apparatus may further include a plurality of pressing members configured to correspond to the plurality of branch flow paths, respectively, and configured to be individually movable. The valve may be provided with a plurality of seating portions disposed on opposite sides to the plurality of protrusions so that the plurality of pressing members are seated on the seating portions, respectively.

In an embodiment, the medicinal liquid flow control apparatus may further include an air pass filter disposed in the inlet flow path or the outlet flow path.

According to an embodiment of the present disclosure, a medical staff such as a doctor is able to conveniently control the flow rate of a medicinal liquid being administered to a patient.

According to an embodiment of the present disclosure, the flow rate of a medicinal liquid being administered to a patient may be controlled to various values.

According to an embodiment of the present disclosure, it is possible to prevent the flow of a medicinal liquid from being stopped by air bubbles in the medicinal liquid transfer tube or to prevent the flow speed of the medicinal liquid from becoming significantly lower than a preset level.

### BRIEF DESCRIPTION OF THE DRAWINGS

FIG. 1 is a perspective view of a medicinal liquid flow control apparatus according to an embodiment of the present disclosure.
FIG. 2 is a plan view of the medicinal liquid flow control apparatus illustrated in FIG. 1.
FIG. 3 is a cross-sectional view taken along line III-III indicated in FIG. 2, illustrating an example of a direction in which a medicinal liquid flows.
FIG. 4 is a cross-sectional view taken along line III-III indicated in FIG. 2, illustrating another example of a direction in which a medicinal liquid flows.
FIG. 5 is an exploded perspective view of the medicinal liquid flow control apparatus illustrated in FIG. 1.
FIG. 6 is an exploded perspective view illustrating the medicinal liquid flow control apparatus illustrated in FIG. 3 from another angle.
FIG. 7 is an exploded perspective view illustrating the medicinal liquid flow control apparatus illustrated in FIG. 3 from yet another angle.
FIG. 8 is a perspective view illustrating a cross section taken along line VIII-VIII indicated in FIG. 2.
FIG. 9 is a cross-sectional view taken along line VIII-VIII indicated in FIG. 2.
FIG. 10 is a bottom perspective view illustrating an actuator illustrated in FIGS. 5 to 7.
FIG. 11 is a perspective view illustrating a valve housing illustrated in FIGS. 5 to 7.
FIG. 12 is a view illustrating an example of flow rate control according to the operation of the medicinal liquid flow control apparatus according to an embodiment of the present disclosure.
FIG. 13 is a view illustrating another example of flow rate control according to the operation of the medicinal liquid flow control apparatus of FIG. 12.
FIG. 14 is a view illustrating yet another example of flow rate control according to the operation of the medicinal liquid flow control apparatus of FIG. 12.
FIG. 15 is a view illustrating still another example of flow rate control according to the operation of the medicinal liquid flow control apparatus of FIG. 12.
FIG. 16 is a view illustrating yet another example of flow rate control according to the operation of the medicinal liquid flow control apparatus of FIG. 12.

### DETAILED DESCRIPTION

Embodiments of the present disclosure are exemplified for the purpose of explaining the technical idea of the present disclosure. The scope of the rights according to the present disclosure is not limited to the embodiments presented below or the detailed descriptions of such embodiments.

All technical and scientific terms used in the present disclosure have the meaning generally understood by those of ordinary skill in the art to which the present disclosure pertains, unless otherwise defined. All terms used in the present disclosure are chosen for the purpose of more clearly describing the present disclosure and are not chosen to limit the scope of the present disclosure.

As used in the present disclosure, expressions such as "comprising," "including," "having," and the like are to be understood as open-ended terms having the possibility of encompassing other embodiments, unless otherwise mentioned in the phrase or sentence containing such expressions.

The singular form described in the present disclosure may include a plural meaning, unless otherwise mentioned. This applies equally to the singular form recited in the claims.

Expressions such as "first" and "second" used in the present disclosure are used to distinguish a plurality of components from each other, and do not limit the order or importance of the corresponding components.

In the present disclosure, where it is mentioned in the present disclosure that one element is "connected" to another element, it is to be understood that said one element may be directly connected to said another element, or may be connected to said another element via a new additional element.

Hereinafter, embodiments of the present disclosure will be described with reference to the accompanying drawings. In the accompanying drawings, like or relevant components are indicated by like reference numerals. In the following description of embodiments, repeated descriptions of the identical or relevant components will be omitted. However, even if a description of a component is omitted, such a component is not intended to be excluded in an embodiment.

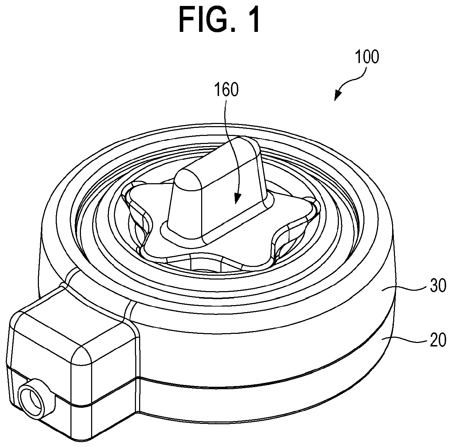

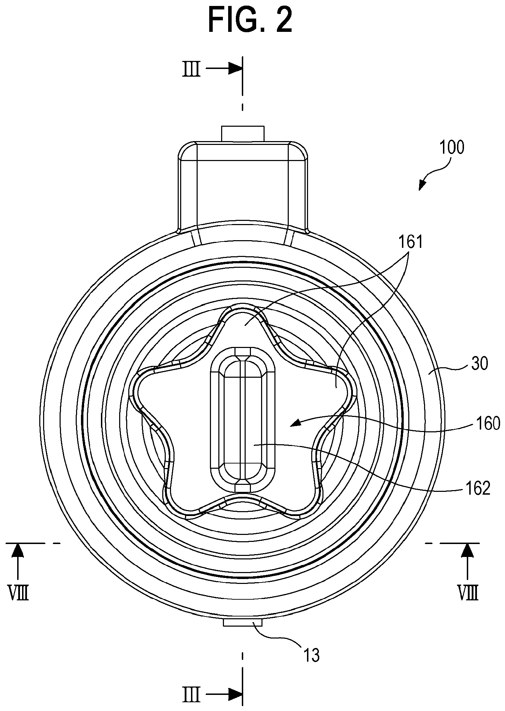

FIG. 1 is a perspective view of a medicinal liquid flow control apparatus according to an embodiment of the present disclosure. FIG. 2 is a plan view of the medicinal liquid flow control apparatus illustrated in FIG. 1.

As illustrated in FIGS. 1 and 2, the medicinal liquid flow control apparatus 100 according to an embodiment of the present disclosure includes a medicinal liquid flow path 10 that guides the flow of a medicinal liquid. Here, the medicinal liquid is a medicinal liquid that can be administered to a patient for treatment, and is in liquid state. The medicinal liquid flow control apparatus 100 is configured to control the flow rate of the medicinal liquid administered to a patient.

The medicinal liquid flow control apparatus 100 may include cases 20 and 30. The medicinal liquid flow control apparatus 100 may include housings 181 and 170 that form at least a portion of the medicinal liquid flow path 10. The cases 20 and 30 may include a first case 20 and a second case 30 coupled to each other. The housings 181 and 170 may include a flow path forming housing 181 and a valve housing 170 coupled to each other. The housings 181 and 170 are provided with a hole 11a which is one end of the medicinal liquid flow path 10 and a hole 13a which is the other end of the medicinal liquid flow path 10, respectively.

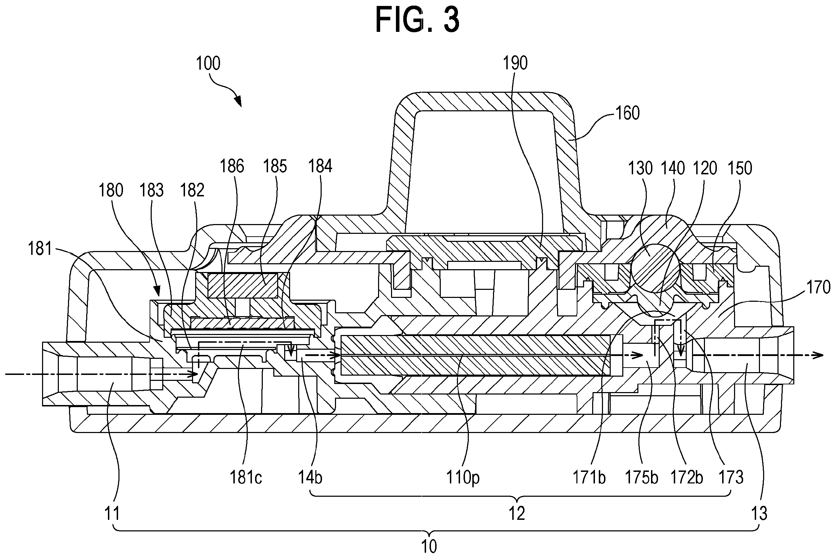

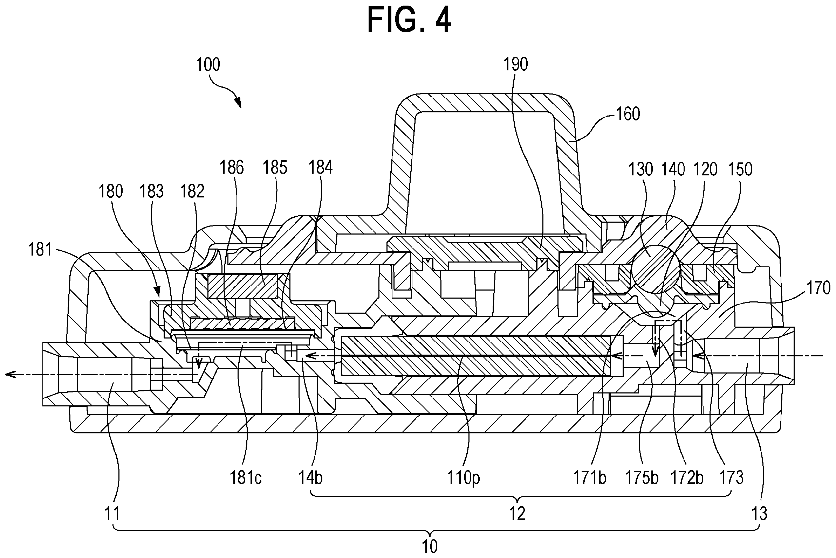

FIG. 3 is a cross-sectional view taken along line III-III indicated in FIG. 2, illustrating an example of a direction in which a medicinal liquid flows. FIG. 4 is a cross-sectional view taken along line III-III indicated in FIG. 2, illustrating another example of a direction in which a medicinal liquid flows.

As illustrated in FIG. 3, the medicinal liquid flow path 10 includes a first flow path 11, a second flow path 13, and a plurality of branch flow paths 12 interconnecting the first flow path 11 and the second flow path 13. In the embodiment illustrated in FIG. 3, the first flow path 11 is an inlet through which a medicinal liquid flows in, and the second flow path 13 is an outlet through which the medicinal liquid flows out. As indicated by arrows in FIG. 3, a medicinal liquid flows in through the first flow path 11, flows via at least one of the plurality of branch flow paths 12, and flows out through the second flow path 13. In the embodiment illustrated in FIG. 4, the second flow path 13 is an inlet through which a medicinal liquid flows in, and the first flow path 11 is an outlet through which the medicinal liquid flows out. As indicated by arrows in FIG. 4, a medicinal liquid flows in through the second flow path 13, flows via at least one of the plurality of branch flow paths 12, and flows out through the first flow path 11. That is, the flow path through which the medicinal liquid flows in may be understood as the inlet, and the flow path through which the medicinal liquid flows out may be understood as the outlet. The plurality of branch flow paths 12 branch from the inlet, and the medicinal liquid joined from the plurality of branch flow paths 12 flows out through the outlet.

Here, the number of branch flow paths may be n. "n" referred to throughout the present specification means "a natural number greater than or equal to 2". The number of pressing members 130 to be described later may be n, and the plurality of branch flow paths may be opened and closed by the pressing members 130.

Referring to FIGS. 3 to 7, the hole 11a provided outside the flow path forming housing 181 may define a portion of the first flow path 11. The first flow path 11 may be provided in a filter mechanism 180 to be described later. In an embodiment, the first flow path 11 may be formed by sequentially connecting the hole 11a and the flow path provided in the filter mechanism 180.

Referring to FIGS. 3 to 7, the hole 13a provided outside the valve housing 170 may define a portion of the second flow path 13. A connection hole 173 to be described later may define a portion of the second flow path 13. In an embodiment, the second flow path 13 may be formed by sequentially connecting the connection hole 173 and the hole 13a.

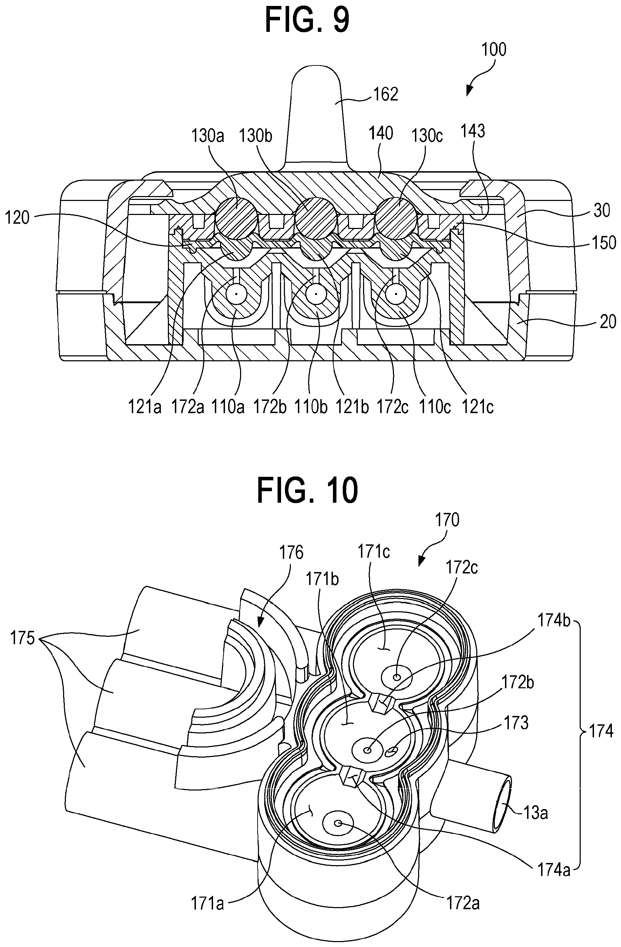

Referring to FIGS. 3 to 7, each of n holes 14a, 14b, and 14c in a sealer 14 may define a portion of a corresponding one of the n branch flow paths. Each of capillary flow paths 110p of the n transfer tubes 110a, 110b, and 110c may define a portion of a corresponding one of the n branch flow paths. Each of n holes 175a, 175b, and 175c provided in the connection tube 175 may define a portion of a corresponding one of the n branch flow paths. Referring to FIG. 10, each of n valve holes 172a, 172b, and 172c may define a portion of a corresponding one of the n branch flow paths. Each of n valve spaces 171a, 171b, and 171c may define a portion of a corresponding one of the n branch flow paths. Each of n-1 communication flow paths 174a and 174b, which will be described later, may define a portion of a corresponding one of "n-1" branch flow paths that are a part of the "n branch flow paths."

In an embodiment, the plurality of branch flow paths 12 may include a first branch flow path, a second branch flow path, and a third branch flow path. For example, the first branch flow path may be formed by sequentially connecting the first hole 14a of the sealer 14, the capillary flow paths 110p provided in the first transfer tube 110a, the first hole 175a of the connection tube 175, the first valve hole 172a, the first valve space 171a, and the first communication flow path 174a to each other. The second branch flow path may be formed by sequentially connecting the second hole 14b of the sealer 14, the capillary flow paths 110p provided in the second transfer tube 110b, the second hole 175b of the connection tube 175, the second valve hole 172b, and the second valve space 171b to each other. The third branch flow path may be formed by sequentially connecting the third hole 14c of the sealer 14, the capillary flow path 110p provided in the third transfer tube 110c, the third hole 175c of the connection tube 175, the third valve hole 172c, the third valve space 171c, and the second communication flow path 174b to each other. In an embodiment in which a plurality of holes are formed in the flow path forming housing 181, each of the plurality holes of the flow path forming housing 181, which correspond to the first hole 14a, the second hole 14b, and the third hole 14c of the sealer 14, respectively, may define a portion of a corresponding one of the first branch flow path, the second branch flow path, and the third branch flow path.

The medicinal liquid flow control apparatus 100 may further include a sealer 14 disposed between the first flow path 11 and the branch flow paths 12. The sealer 14 may be formed of a material such as rubber. For example, the sealer 14 may be a rubber packing, and a flow path branching from the first flow path 11 into the branch flow paths 12 may be provided in the sealer 14. The medicinal liquid may be guided to pass through the capillary flow paths 110p of the branch flow paths 12 through the sealer 14.

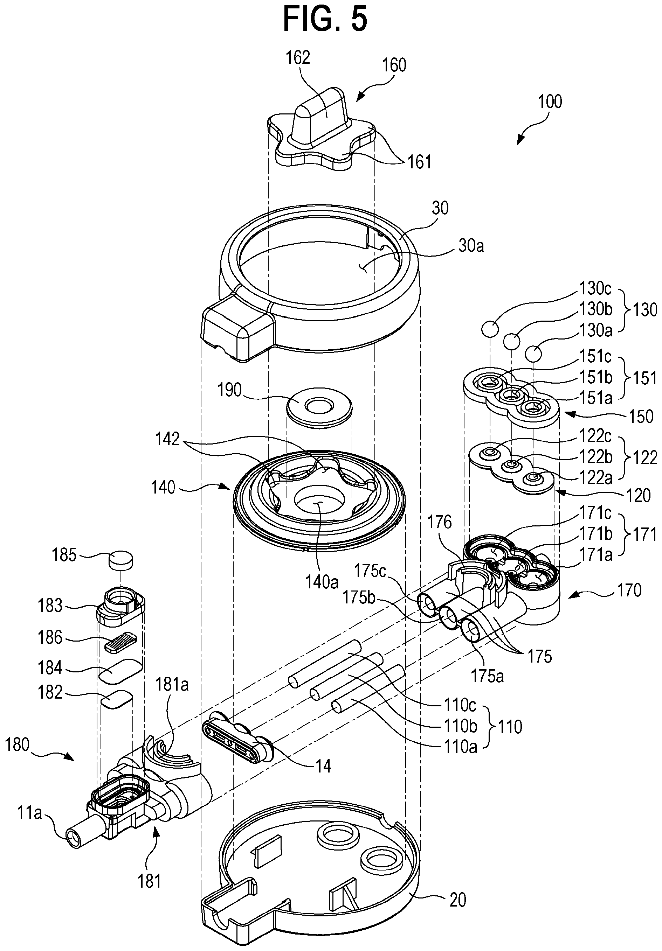

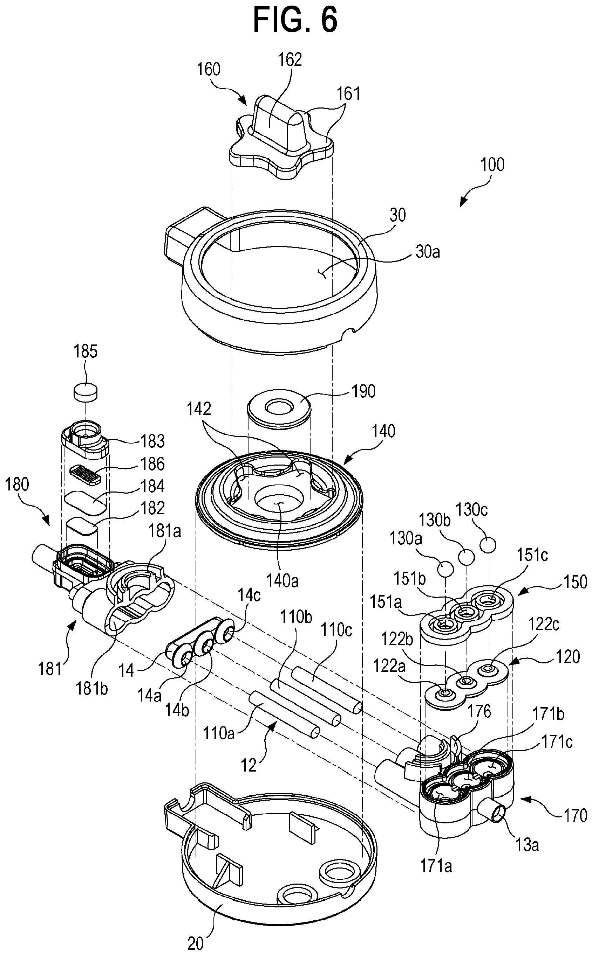

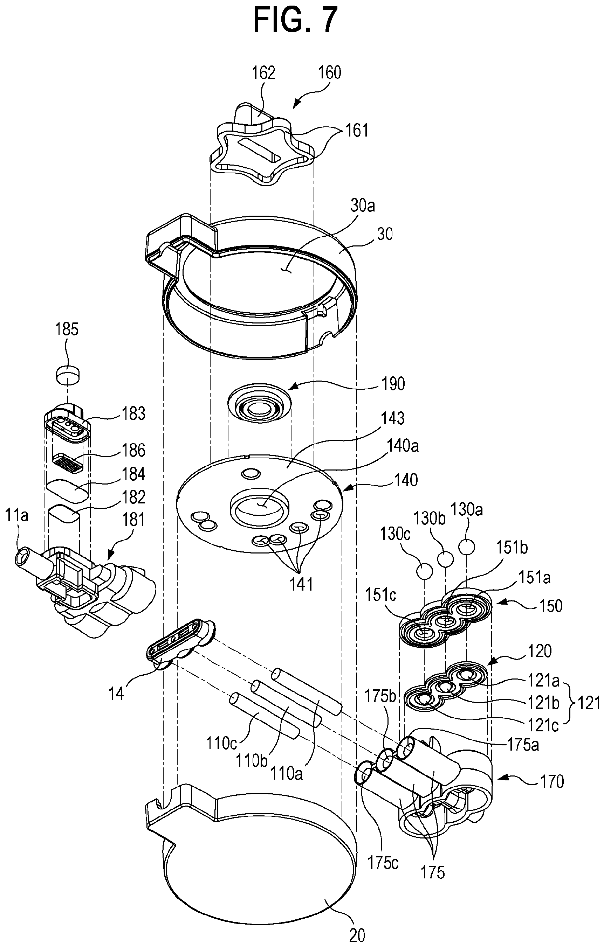

FIG. 5 is an exploded perspective view of the medicinal liquid flow control apparatus illustrated in FIG. 1. FIG. 6 is an exploded perspective view illustrating the medicinal liquid flow control apparatus illustrated in FIG. 3 from another angle. FIG. 7 is an exploded perspective view illustrating the medicinal liquid flow control apparatus illustrated in FIG. 3 from yet another angle.

As illustrated in FIGS. 5 to 7, the medicinal liquid flow control apparatus 100 in an embodiment includes a plurality of transfer tubes 110 and a valve 120.

The plurality of transfer tubes 110 are configured to correspond to the plurality of branch flow paths 12, respectively. The plurality of transfer tubes 110 provide the capillary flow paths 110p defining at least a portion of a corresponding one of the plurality of branch flow paths 12. The transfer tube 110 may include a polymer microtube. The number of transfer tubes 110 may be n. The n transfer tubes 110 may include capillary flow paths 110p having different inner diameters (or cross-sectional areas). The number of transfer tubes 110 may be variously determined based on the precision in controling a flow rate via the medicinal liquid flow control apparatus 100. In addition, the length and/or inner diameter of each of the capillary flow paths 110p corresponding to the plurality of transfer tubes 110 may be determined differently based on the precision in controling a flow rate via the medicinal liquid flow control apparatus 100. For example, the plurality of transfer tubes 110 may have an inner diameter of about 0.04 mm to 0.08 mm. Accordingly, by selectively opening/closing the plurality of capillary flow paths 110p which are set to different flow rates, the flow rate control may be facilitated and may be controlled to various flow rates. In an embodiment, the plurality of transfer tubes 110 may include at least two transfer tubes configured to allow the medicinal liquid to flow at different flow rates. In an embodiment to be described below, the plurality of branch flow paths 12 include a first branch flow path, a second branch flow path, and a third branch flow path, and the plurality of transfer tubes 110 include a first transfer tube 110a, a second transfer tube 110b, and a third transfer tube 110c. For example, the first transfer tube 110a, the second transfer tube 110b, and the third transfer tube 110c may be configured such that a medicinal liquids of 1.5 cc, 1.0 cc, and 0.5 cc pass therethrough in an hour, respectively.

The valve 120 is configured to control the flow rate of the medicinal liquid flowing out through the second flow path 13 by selectively opening and closing the plurality of branch flow paths 12. For example, the valve 120 functions as a valve mechanism interconnecting the branch flow path 12 and the second flow path 13.

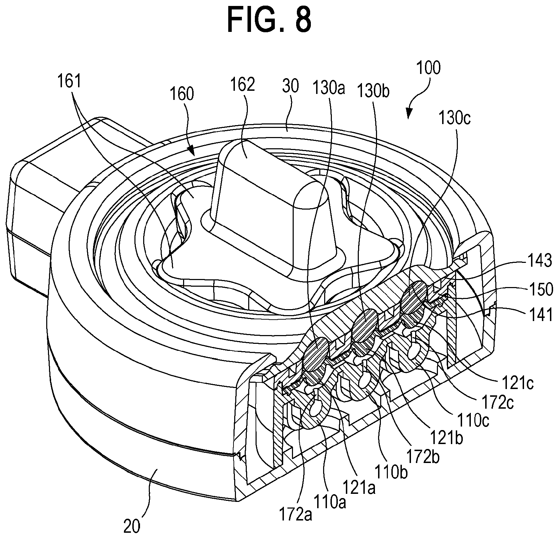

FIG. 8 is a perspective view illustrating a cross section taken along line VIII-VIII indicated in FIG. 2. FIG. 9 is a cross-sectional view taken along line VIII-VIII indicated in FIG. 2.

As illustrated in FIGS. 8 and 9, in an embodiment, the medicinal liquid flow control apparatus 100 may further include a plurality of pressing members 130 configured to correspond to the plurality of branch flow paths 12, respectively. The plurality of pressing members 130 may be configured to be individually movable. The pressing members 130 have a spherical shape and are configured such that relative distances thereof with respect to the valve 120 vary as they move along a direction (e.g., the vertical direction UD in FIG. 3) perpendicular to the longitudinal direction RL of the plurality of transfer tubes 110 (i.e., branch flow paths). As another example, the pressing members 130 may have a bar shape in which both ends have a hemispherical shape. For example, the plurality of pressing members 130 may include a first pressing member 130a, a second pressing member 130b, and a third pressing member 130c, which correspond to the first transfer tube 110a, the second transfer tube 110b, and the third transfer tube 110c, respectively.

In an embodiment, the medicinal liquid flow control apparatus 100 may further include an actuator 140 configured to move the plurality of pressing members 130. The actuator 140 may be located on the opposite side to the valve 120 with respect to the pressing member 130. The actuator 140 may have a plate shape and be configured to rotate about a rotation center following a direction (e.g., the vertical direction UD) perpendicular to the longitudinal direction RL of the plurality of transfer tubes 110. As the actuator 140 rotates, at least one of the plurality of pressing members 130 may move relatively with respect to the valve 120. Although the actuator 140 is described and illustrated as having a disk shape in this embodiment, embodiments of the disclosure are not limited thereto. For example, the actuator 140 may have a polygonal plate shape.

FIG. 10 is a bottom perspective view illustrating an actuator shown in FIGS. 5 to 7.

As illustrated in FIG. 10, in an embodiment, a plurality of recesses 141 may be provided in the actuator 140. The plurality of recesses 141 may be spaced apart from each other. The plurality of recesses 141 may be configured to accommodate the plurality of pressing members 130. The valve 120 may be configured to be elastically restored and press the pressing members 130 in the recessed direction of the recesses 141 (e.g., upward). The plurality of recesses 141 may be provided on the bottom surface of the actuator 140. The plurality of recesses 141 may be concave in the direction opposite to the valve 120 with reference to the pressing member 130 (e.g., upward). The plurality of recesses 141 may be arranged to be spaced apart from each other along the circumferential direction about the center of rotation. The number and provided positions of recesses 141 may be determined based on the number of transfer tubes 110 and/or the precision in controling a flow rate via the medicinal liquid flow control apparatus 100.

In an embodiment, the actuator 140 is configured to be actuated in a way that the recesses 141 move in a direction transverse to the recessed direction thereof. As the actuator 140 is actuated, the plurality of pressing members 130 may be selectively accommodated in the plurality of recesses 141. The recessed direction of the recesses 141 may mean the upward direction in the up-down direction UD, and the direction transverse to the recessed direction of the recesses 141 may mean a direction following a plane defined by a longitudinal direction RL and a front-rear direction FR in which the plurality of transfer tubes 110 are arranged. That is, the plate-shaped actuator 140 may be configured such that as the actuator 140 rotates about the center of rotation, the plurality of pressing members 130 are selectively accommodated in the plurality of recesses 141 to open and close the plurality of branch flow paths 12.

In an embodiment, the valve 120 may be configured to open the corresponding branch flow paths 12 in the state in which the pressing members 130 are accommodated in the recesses 141. That is, the valve 120 is configured to open the branch flow paths 12 corresponding to the pressing members 130 accommodated in the recesses 141 among the plurality of pressing members 130. Conversely, the valve 120 may be configured to close the branch flow paths 12 corresponding to the pressing members 130 not accommodated in the recesses 141. For example, when the pressing members 130 are accommodated in the recesses 141, the valve 120 is released so that the branch flow paths 12 are opened, and when the pressing members 130 are not accommodated in the recesses 141, the valve 120 is pressed and the branch flow paths 12 are closed.

In an embodiment, the medicinal liquid flow control apparatus 100 may further include a guide member 150 disposed between the valve 120 and the plurality of pressing members 130. The guide member 150 may be configured to guide the movement of the plurality of pressing members 130. The guide member 150 is configured to cover the valve 120 and is coupled to the valve housing 170. The guide member 150 may be configured to guide the pressing members 130 to move in the up-down direction UD and space the plurality of pressing members 130 apart from each other in the front-rear direction FR. To this end, the guide member 150 may include a plurality of guide holes 151 which are spaced apart from each other in the front-rear direction FR and configured to accommodate at least a portion of the plurality of pressing members 130. The guide member 150 may have a thickness in the up-down direction UD equal to or larger than the distance in which the plurality of pressing members 130 move in the up-down direction UD between the pressed state and the released state of the valve 120. The number of guide holes 151 may be n. For example, the plurality of guide holes 151 may include a first guide hole 151a, a second guide hole 151b, and a third guide hole 151c, which correspond to the first pressing member 130a, the second pressing member 130b, and the third pressing member 130c, respectively.

In an embodiment, the medicinal liquid flow control apparatus 100 may further include an actuator 140 and an operation member 160. As described above, the actuator 140 may be configured to move the pressing members 130 or may be configured to actuate the valve 120. The operation member 160 is configured to actuate the actuator 140 in the state of being engaged with the actuator 140. For example, the operation member 160 may be configured to control a flow rate of the medicinal liquid by rotating the actuator 140. A shaft hole 140a for supporting rotation of the actuator 140 is provided in the center of the actuator 140.

In an embodiment, the operation member 160 may be provided with an engaging portion 161, and the actuator 140 may be provided with a counterpart engaging portion 142 that accommodates and is engaged with the engaging portion 161. That is, the counterpart engaging portion 142 may be formed to be concave to accommodate the engaging portion 161. The engaging portion 161 and the counterpart engaging portion 142 may have complementary shapes. For example, the engaging portion 161 may have a star-shaped or polygonal planar shape, and the counterpart engaging portion 142 may be concave downward to correspond to the planar shape of the engaging portion 161. The operation member 160 may further include a handle portion 162 protruding upward from the engaging portion 161. A user may easily rotate the actuator 140 by holding and rotating the handle portion 162.

In an embodiment, the operation member 160 may be removably coupled to a portion of the actuator 140.. For example, a doctor in charge or a nurse in charge may carry the operation member 160 or store the operation member 160 in a particular place and may use the operation member 160 by coupling the operation member 160 to the medicinal liquid flow control apparatus 100 to control the flow rate of the medicinal liquid. Accordingly, it is possible to prevent a patient or an unauthorized user from arbitrarily operating the operation member 160 to control the flow rate of the medicinal liquid. That is, the operation member 160 may function as a key that is coupled to the medicinal liquid flow control apparatus 100 to rotate the operating member 140 when it is necessary to control the flow rate of the medicinal liquid.

In an embodiment, the valve 120 may be configured to be elastically deformed by being pressed and elastically restored by being released. For example, the valve 120 may be made of a material that can be elastically deformed and elastically restored, such as a rubber material. In this embodiment, the valve 120 may be configured to be elastically deformed by the pressing of the pressing members 130, thereby closing the corresponding branch flow paths 12, and may be configured to provide an elastic restoration force to move the pressing members 130 upward when the pressing members 130 are accommodated in the recesses 141 of the actuator 140, thereby opening the corresponding branch flow paths 12.

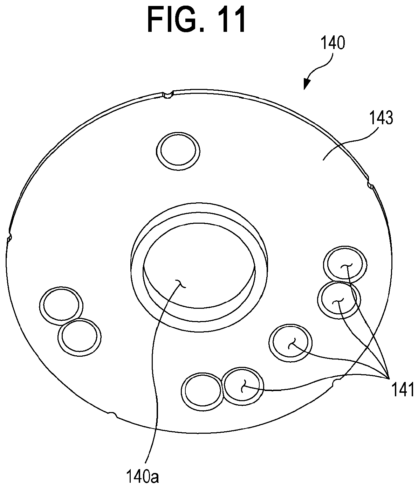

FIG. 11 is a perspective view illustrating a valve housing illustrated in FIGS. 5 to 7.

As illustrated in FIG. 11, in an embodiment, the medicinal liquid flow control apparatus 100 may further include a valve housing 170 that is provided with a plurality of valve spaces 171. The plurality of valve spaces 171 are configured to be covered by the valve 120. The valve 120 functions as a cover or a sealing member that prevents the medicinal liquid held in the valve spaces 171 from flowing upward. Each of the plurality of valve spaces 171 defines a portion of a corresponding one of the plurality of branch flow paths 12. The plurality of valve spaces 171 are formed to be concave downward and are arranged in a line in the front-rear direction FR. That is, it may be understood that the plurality of concave valve spaces 171 are closed by the valve 120 as a sealing member and are respectively included in a portion of the plurality of the branch flow paths 12. The number of valve spaces 171 may be n. For example, the plurality of valve spaces 171 may include a first valve space 171a, a second pressing member 171b, and a third valve space 171c, which correspond to the first transfer tube 110a, the second transfer tube 110b, and the third transfer tube 110c, respectively.

The valve housing 170 may include a plurality of connection tubes 175 to which the plurality of transfer tubes 110 forming the capillary flow paths 110p are connected. The plurality of transfer tubes 110 may be inserted into the plurality of connection tubes 175 or the plurality of connection tubes 175 may be inserted into the plurality of transfer tubes 110. The number of connection tubes 175 may be n. For example, the plurality of connection tubes 175 may include a first hole 175a, a second hole 175b, and a third hole 175c, into which the first transfer tube 110a, the second transfer tube 110b, and the third transfer tube 110c are inserted, respectively.

In an embodiment, the valve housing 170 may be provided with a plurality of valve holes 172 that allow the plurality of valve spaces 171 to communicate with the capillary flow paths 110p of the plurality of transfer tubes 110, respectively. The plurality of valve holes 172 may be provided in the bottom portions of the plurality of valve spaces 171 corresponding thereto. The plurality of valve holes 172 communicate with the plurality of connection tubes 175, respectively. The medicinal liquid flowing in through the plurality of connection tubes 175 may be temporarily held in the valve spaces 171 through the valve holes 172. The medicinal liquid temporarily held in the valve spaces 171 may flow into the plurality of connection tubes 175 through the valve holes 172. The number of valve holes 172 may be n. For example, the plurality of valve spaces 172 may include a first valve hole 172a, a second valve hole 172b, and a third valve space 172c, which correspond to the first transfer tube 110a, the second transfer tube 110b, and the third transfer tube 110c, respectively. In this case, each of the first valve hole 172a, the second valve hole 172b, and the third valve hole 172c may be opened or closed by the elastic deformation of the valve 120. Accordingly, it is possible to control the flow rate of the medicinal liquid in the medicinal liquid flow path 10.

In an embodiment, the valve 120 may be configured to close the valve holes 172 when pressed by the pressing members 130. For example, in the case in which the valve 120 is made of an elastically deformable material such as rubber, when the pressing members 130 move downward to press the valve 120 downward, the valve 120 is elastically deformed in the valve spaces 171. Accordingly, the elastically deformed portions of the valve 120 may come into contact with the valve holes 172 and close the valve holes 172.

In an embodiment, one of the plurality of valve spaces 171 may be provided with a connection hole 173 to which the second flow path 13 is connected. The plurality of valve spaces 171 communicate with the second flow path 13 through the connection hole 173, and the plurality of branch flow paths 12 join through the connection hole 173. The medicinal liquid flowing in from the second flow path 13 is temporarily held in the valve spaces 171 through the connection hole 173, or the medicinal liquid temporarily held in the valve spaces 171 flows into the second flow path 13 through the connection hole 173.

In an embodiment, a communication flow path 174 for communicating two valve spaces 171 adjacent to each other among the plurality of valve spaces 171 may be provided. The plurality of valve holes 172 and the connection hole 173 communicate with each other through the communication flow path 174. That is, the valve holes 172 of the valve spaces 171 in which the connection hole 173 is not provided communicate with the connection hole 173 via the communication flow path 174, and the valve hole 172 of the valve space 171 in which the connection hole 173 is provided directly communicates with the connection hole 173 without passing through the communication flow path 174. The number of communication flow paths 174 may be n-1. In an embodiment, the communication flow path 174 may be provided by covering a groove provided in the valve housing 170 with the valve 120. In another embodiment (not illustrated), the communication flow path 174 may be configured with a hole provided in the valve housing 170. The medicinal liquid located in each of the n valve spaces 171a, 171b, and 171c may be collected into one valve space 171b by the communication flow path 174. In an embodiment, the communication flow path 174 may include a first communication flow path 174a configured to interconnect the first valve space 171a and the second valve space 171b and a second communication flow path 174b configured to interconnect the second valve space 171b and the third valve space 171c.

In an embodiment, the connection hole 173 and the communication flow path 174 may be configured to maintain the opened state in a state in which the valve 120 opens the valve holes 172 and in a state in which the valve 120 closes the valve holes 172. That is, the connection hole 173 and the communication flow path 174 are configured to always maintain a communication with each other regardless of the open or closed state of the valve holes 172 by valve 120. Accordingly, in the state in which the valve holes 172 of the valve spaces 171 in which the connection hole 173 is not provided are closed or opened, the valve holes 172 of the valve spaces 171 in which the connection hole 173 is not provided may communicate with the connection hole 173 through the communication flow path 174. As a result, the medicinal liquid is flowable along paths that communicate with each other.

In an embodiment, the valve 120 may include a plurality of protrusions 121 that protrude toward the plurality of valve spaces 171 and are configured to close the corresponding branch flow paths 12, respectively. At least some of the plurality of protrusions 121 protrude toward the plurality of valve spaces 171 and are accommodated in the valve spaces 171. Accordingly, by reducing the moving distance in the up-down direction UD of the pressing members 130, it is possible to miniaturize the medicinal liquid flow control apparatus 100. In addition, since the flow rate of the medicinal liquid temporarily held by the valve spaces 171 can be maintained low due to the plurality of protrusions 121, it is possible to shorten the initial time for administering the medicinal liquid. In addition, by reducing the flow rate of the medicinal liquid remaining in the medicinal liquid flow control apparatus 100 after the administration of the medicinal liquid is terminated, it is possible to reduce the amount of the medicinal liquid that is not used and discarded. The number of protrusions 121 may be n. For example, the plurality of protrusions 121 may include a first protrusion 121a, a second protrusion 121b, and a third protrusion 121c, which correspond to the first pressing member 130a, the second pressing member 130b, and the third pressing member 130c, respectively. In this case, each of the first protrusion 121a, the second protrusion 121b, and the third protrusion 121c may be configured to open or close the first valve hole 172a, the second valve hole 172b, and the third valve hole 172c, respectively.

In an embodiment, the valve 120 may be provided with a plurality of seating portions 122 disposed on opposite sides to the plurality of protrusions 121, wherein the plurality of pressing members 130 are seated on the valve 120. Since the plurality of pressing members 130 are seated on the plurality of seating portions 122, each of the plurality of pressing members 130 is configured to be accurately positioned in a corresponding one of the plurality of valve spaces 171, so that the valve holes 172 are securely closed. The plurality of seating portions 122 may be concave in the downward direction. The radius of curvature of the plurality of pressing members 130 may be configured to match the radius of curvature of the plurality of seating portions 122 so that the plurality of pressing members 130 can be accurately seated on the plurality of seating portions 122. The number of seating portions 122 may be n. For example, the plurality of seating portions 122 may include a first seating portion 122a, a second seating portion 122b, and a third seating portion 122c, which correspond to the first pressing member 130a, the second pressing member 130b, and the third pressing member 130c, respectively.

In an embodiment, the medicinal liquid flow control apparatus 100 may include a first case 20 and a second case 30 configured to cover the plurality of transfer tubes 110 and the valve 120. The first case 20 and the second case 30 may be configured to be coupled to each other. The plurality of transfer tubes 110 and the valve housing 170 are fixed to the first case 20. The first flow path 11 and the second flow path 13 are configured to protrude outward from the first case 20 and the second case 30 and are fixed by the first case 20 and the second case 30. The second case 30 may be provided with an operating hole 30a such that the operation member 160 is exposed. After the operation member 160 is inserted through the operation hole 30a and coupled to the actuator 140, the position of the actuator 140 may be adjusted. For example, the actuator 140 may be configured to be changed in its position by rotation, or may be configured to be changed in its position by a movement other than rotation (e.g., sliding).

In an embodiment, the medicinal liquid flow control apparatus may further include an air pass filter (e.g., the filter mechanism 180) disposed at the first flow path 11. The filter mechanism 180 removes the gas contained in the medicinal liquid flowing in the medicinal liquid flow path 10 and the residual gas dissolved in the medicinal liquid. This prevents clogging of the capillary flow paths by micro air bubbles.

Referring back to FIGS. 5 to 7, the filter mechanism 180 according to an embodiment includes a flow path forming housing 181, a boundary filter 182, a filter cap 183, and at least one air pass filter. In an embodiment in which the filter mechanism 180 is provided, the flow path forming housing 181 may be referred to as a filter housing.

The flow path forming housing 181 may be disposed on the medicinal liquid flow path 10 and form at least a portion of the first flow path 11. The medicinal liquid flowing in through the first flow path 11 (e.g., the embodiment of FIG. 3) or the medicinal liquid flowing out through the first flow path 11 (e.g., the embodiment of FIG. 4) flows through the flow path forming housing 181. The flow path forming housing 181 may be provided with an inner hole 181b. A portion in which n branch flow paths start may be provided inside the inner hole 181b, and a portion in which n branch flow paths start may be configured by the sealer 14. The sealer 14 may be inserted into the inner hole 181b.

The first flow path 11 may include a filter flow path 181c. The filter flow path 181c may be disposed inside the flow path forming housing 181. In this case, the medicinal liquid may flow sequentially through the hole 11a and the filter flow path 181c or sequentially flow through the filter flow path 181c and the hole 11a.

The boundary filter 182 may be disposed on the first flow path 11. The boundary filter 182 may be disposed in the flow path forming housing 181. The boundary filter 182 may be made of a hydrophilic material. The boundary filter 182 is configured to act as a pressure interface between an upstream flow path portion and a downstream flow path portion with respect to the boundary filter 182 when the boundary filter 182 is wet with the medicinal liquid. Due to the boundary filter 182, the upstream flow path portion and the downstream flow path portion may have different internal pressures. For example, the boundary filter 182 may be configured in at least one of a mesh structure and a fiber structure. The boundary filter 182 may be configured to filter impurities.

The filter cap 183 is coupled to the flow path forming housing 181 in an airtight and watertight manner. The filter cap 183 provides a passage through which the air contained in the medicinal liquid flowing in through the first flow path 11 flows out. The filter cap 183 may provide at least one vent hole (not illustrated) located at a point where an air passage connected to the external space communicates.

The at least one air pass filter may be formed of a hydrophobic material and configured to filter the air in the medicinal liquid flow path 10. The air pass filter may be configured to block the medicinal liquid and allow the air to pass therethrough. The air pass filter may be disposed on the filter cap 183. The air pass filter may include a first air pass filter 184 disposed at a boundary between the air passage and the filter flow path 181c. The air pass filter may further include a second air pass filter 185 disposed on the air passage.

For example, the air pass filter may include a first air pass filter 184 and a second air pass filter 185 that are sequentially disposed on the air passage directed to the filter cap 183 from the flow path forming housing 181. The first air pass filter 184 may be disposed inside the filter cap 183, and the second air pass filter 185 may be disposed on the opposite side to the first air pass filter 184 with reference to the filter cap 183. The second air pass filter 185 is configured to allow air that has passed through the first air pass filter 184 to pass therethrough. Therefore, the second air pass filter 185 serves to prevent the internal medicinal liquid from flowing to the exterior even when the pores or bonded portions of the first air pass filter 184 are damaged.

The second air pass filter 185 may be formed of the same material as the first air pass filter 184 or a porous plastic material. As an example, the second air pass filter 185 may be configured such that a hydrophobic porous plastic resin material fills cross-sectional areas of at least a portion of the air passages. For example, the material of the second air pass filter 185 is available from Porex Corporation (web site: https://www.porex.com) of Fairburn, GA 30213, USA. A product released under the name of Porex Hydrophobic Vents from Porex Corporation may be used, wherein the product is made of a material of polyethyl polytetrafluoroethylene.

A filter spacer 186 may be disposed inside the filter cap 183 to be in contact with the first air pass filter 184. The filter spacer 186 is configured to suppress or prevent a phenomenon in which the first air pass filter 184 is bent toward the flow path forming housing 181 by the pressure of the medicinal liquid. A plurality of protrusions may be provided on the surface of the filter spacer 186 facing the first air pass filter 184 (e.g., the surface facing the flow path forming housing 181). Accordingly, the air that has passed through the first air pass filter 184 may flow through the gaps between the plurality of protrusions. For example, the protrusions of the filter spacer 186 may have an embossed shape or a pleated shape. In addition, a plurality of protrusions having an embossed shape or a pleated shape may also be provided on the surface (e.g., the surface facing the filter cap 183) opposite to the surface of the filter spacer 186 facing the first air pass filter 184.

The filter cap 183 may be provided with a recess into which the filter spacer 186 is inserted. The filter cap 183 may include a protrusion that separates the side surface of the filter spacer 186 from the inner surface of the filter cap 183. The filter cap 183 may include a protrusion that separates the rear surface of the filter spacer 186 from the inner surface of the filter cap 183. Through this, air may smoothly flow between the filter spacer 186 and the inner surface of the filter cap 183. The filter cap 183 may be provided with an air hole constituting a portion of an air passage.

In an embodiment, the medicinal liquid flow control apparatus 100 may further include a shaft 190. The shaft 190 may be configured to support the actuator 140 to rotate with respect to the valve housing 170 and the flow path forming housing 181. To this end, the valve housing 170 may be provided with a first shaft guide 176, and the flow path forming housing 181 may be provided with a second shaft guide 181a. The first shaft guide 176 and the second shaft guide 181a are coupled to each other to provide a circular groove. A portion of the shaft 190 passes through the shaft hole 140a of the actuator 140 and is coupled to the first shaft guide 176 and the second shaft guide 181a. The first shaft guide 176 and the second shaft guide 181a may have complementary shapes such that the shaft guides are engaged with each other to be relatively rotatable.

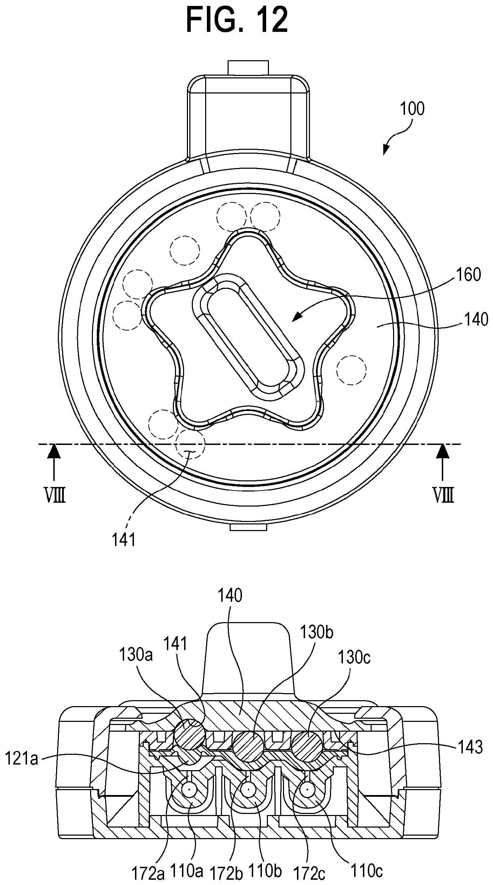

FIGS. 12 to 16 are views illustrating an example of flow rate control according to the operation of the medicinal liquid flow control apparatus according to an embodiment of the present disclosure. FIGS. 12 to 16 each illustrate a plan view and a cross-sectional view according to the operation of the medicinal liquid flow control apparatus, in which the plurality of recesses 141 provided on the bottom surface of the actuator 140 are illustrated by hidden lines around the operation member 160.

Hereinafter, a flow rate control method of the medicinal liquid flow control apparatus according to an embodiment of the present disclosure will be described with reference to FIGS. 12 to 16. In addition, a case where the first transfer tube 110a, the second transfer tube 110b, and the third transfer tube 110c are respectively configured to pass 1.5 cc, 1.0 cc, and 0.5 cc of a medicinal liquid therethrough per hour will be described as an example.

When the operation member 160 is positioned as illustrated in FIG. 12, the first pressing member 130a is accommodated in a recess 141 of the actuator 140, and the second pressing member 130b and the third pressing member 130c are in contact with the bottom surface 143 of the actuator 140. The first protrusion 121a is spaced apart from the first valve hole 172a, so that the first valve hole 172a is opened. The second pressing member 130b and the third pressing member 130c elastically deform the second protrusion 121b and the third protrusion 121c of the valve 120, so that the second valve hole 172b and the third valve hole 172c are closed. Accordingly, the first transfer tube 110a communicates with the connection hole 173 through the opened first valve hole 172a. As a result, the flow rate of the medicinal liquid that can flow through the medicinal liquid flow control apparatus 100 is 0.5 cc per hour which corresponds to the flow rate of the first transfer tube 110a.

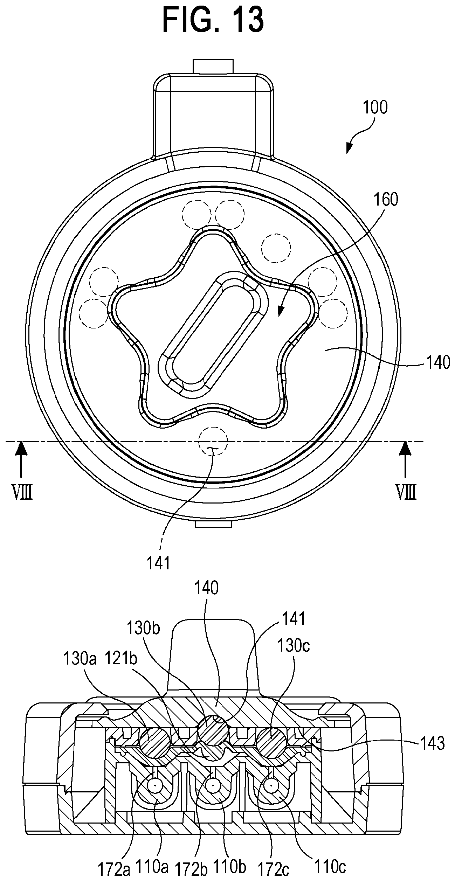

When the operation member 160 is positioned as illustrated in FIG. 13, the second pressing member 130b is accommodated in a recess 141 of the actuator 140, and the first pressing member 130a and the third pressing member 130c are in contact with the bottom surface 143 of the actuator 140. The second protrusion 121b of the valve 120 is spaced apart from the second valve hole 172b, so that the second valve hole 172b is opened. The first pressing member 130a and the third pressing member 130c elastically deform the first protrusion 121a and the third protrusion 121c of the valve 120, so that the first valve hole 172a and the third valve hole 172c are closed. Accordingly, the second transfer tube 110b communicates with the connection hole 173 through the opened second valve hole 172b. As a result, the flow rate of the medicinal liquid that can flow through the medicinal liquid flow control apparatus 100 is 1.0 cc per hour which corresponds to the flow rate of the second transfer tube 110b.

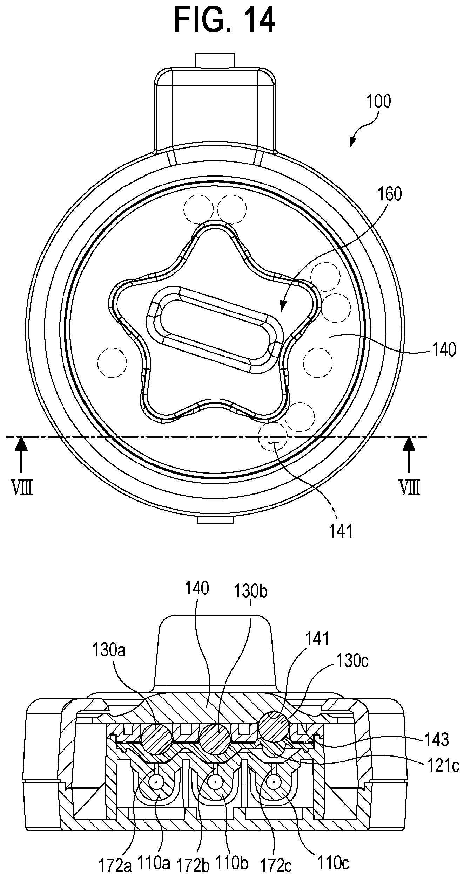

When the operation member 160 is positioned as illustrated in FIG. 14, the third pressing member 130c is accommodated in a recess 141 of the actuator 140, and the first pressing member 130a and the second pressing member 130b are in contact with the bottom surface 143 of the actuator 140. The third protrusion 121c of the valve 120 is spaced apart from the third valve hole 172c, so that the third valve hole 172c is opened. The first pressing member 130a and the second pressing member 130b elastically deform the first protrusion 121a and the second protrusion 121b of the valve 120, so that the first valve hole 172a and the second valve hole 172b are closed. Accordingly, the third transfer tube 110c communicates with the connection hole 173 through the opened third valve hole 172c. As a result, the flow rate of the medicinal liquid that can flow through the medicinal liquid flow control apparatus 100 is 1.5 cc per hour which corresponds to the flow rate of the third transfer tube 110c.

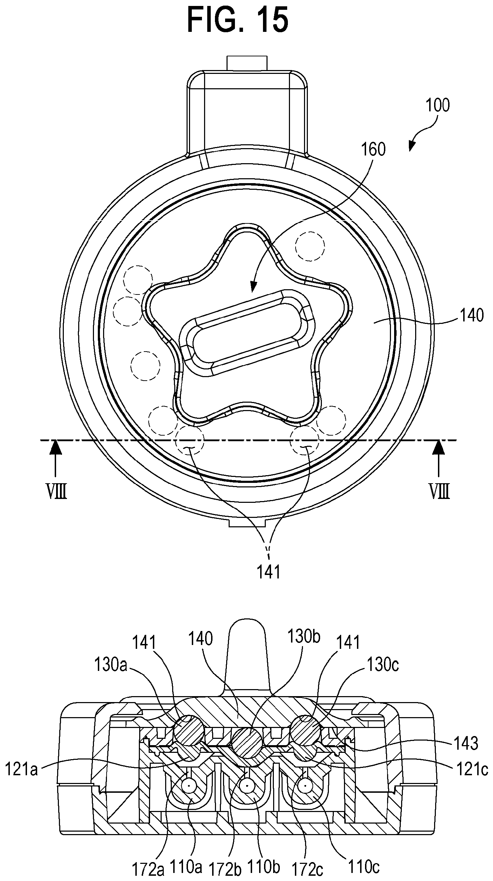

When the operation member 160 is positioned as illustrated in FIG. 15, the first pressing member 130a and the third pressing member 130c are accommodated in recesses 141 of the actuator 140, and the second pressing member 130b is in contact with the bottom surface 143 of the actuator 140. Each of the first protrusion 121a and the third protrusion 121c of the valve 120 is spaced apart from a corresponding one of the first valve hole 172a and the third valve hole 172c, so that the first valve hole 172a and the third valve hole 172c are opened. The second pressing member 130b elastically deforms the second protrusion 121b of the valve 120, so that the second valve hole 172b is closed. Therefore, each of the first transfer tube 110a and the third transfer tube 110c communicates with the connection hole 173 through a corresponding one of the opened first valve hole 172a and third valve hole 172c. As a result, the flow rate of the medicinal liquid that can flow through the medicinal liquid flow control apparatus 100 is 2.0 cc per hour which corresponds to the total flow rate of the first transfer tube 110a and the third transfer tube 110c.

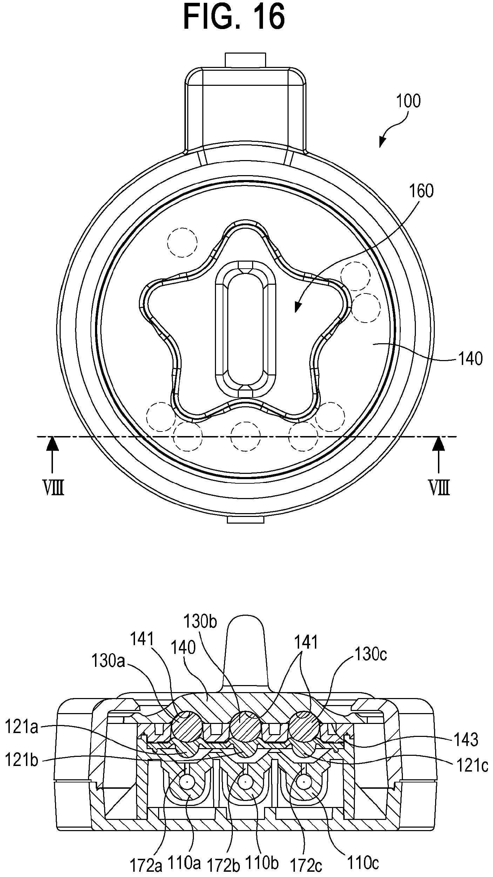

When the operation member 160 is positioned as illustrated in FIG. 16, the first pressing member 130a, the second pressing member 130b, and the third pressing member 130c are accommodated in the recesses 141 of the actuator 140. Each of the first protrusion 121a, the second protrusion 121b, and the third protrusion 121c of the valve 120 is spaced apart from a corresponding one of the first valve hole 172a, the second valve hole 172b, and the third valve hole 172c, so that the first valve hole 172a, the second valve hole 172b, and the third valve hole 172c are opened. Therefore, each of the first transfer tube 110a, the second transfer tube 110b, and the third transfer tube 110c communicates with the connection hole 173 through a corresponding one of the opened first valve hole 172a, second valve hole 172b, and third valve hole 172c. As a result, the flow rate of the medicinal liquid that can flow through the medicinal liquid flow control apparatus 100 is 3.0 cc per hour which corresponds to the total flow rate of the first transfer tube 110a, the second transfer tube 110b, and the third transfer tube 110c.

The technical idea of the present disclosure has been described heretofore with reference to some embodiments and examples illustrated in the accompanying drawings. However, it is to be understood that various substitutions, modifications and alterations may be made without departing from the technical idea and scope of the present disclosure that can be understood by those of ordinary skill in the technical field to which the present disclosure pertains. Further, it is to be understood that such substitutions, modifications and alterations fall within the appended claims.

## Claims

1. A medicinal liquid flow control apparatus comprising a medicinal liquid flow path that guides a flow of a medicinal liquid,
wherein the medicinal liquid flow path includes an inlet flow path through which the medicinal liquid is introduced, a plurality of branch flow paths branching from the inlet flow path, and an outlet flow path to which the plurality of branch flow paths join and through which the medicinal liquid flows out, and
wherein the medicinal liquid flow control apparatus comprises:
a plurality of transfer tubes configured to correspond to the plurality of branch flow paths, respectively, each of the transfer tubes forming a capillary flow path constituting at least a portion of a corresponding one of the plurality of branch flow paths; and
a valve configured to control a flow rate of the medicinal liquid flowing out through the outlet flow path by selectively opening and closing the plurality of branch flow paths.

2. The medicinal liquid flow control apparatus of Claim 1, wherein the plurality of transfer tubes include at least two transfer tubes configured to allow the medicinal liquid to flow at different flow rates.

3. The medicinal liquid flow control apparatus of Claim 1, further comprising:
a plurality of pressing members configured to correspond to the plurality of branch flow paths, respectively, and configured to be individually movable,
wherein the valve is configured to selectively open and close the plurality of branch flow paths by being pressed or released in accordance with a movement of the pressing members.

4. The medicinal liquid flow control apparatus of Claim 3, further comprising:
an actuator configured to move the pressing members.

5. The medicinal liquid flow control apparatus of Claim 4, wherein the actuator is provided with a plurality of recesses to be spaced apart from each other such that the plurality of pressing members are accommodated in the plurality of recesses, respectively, and
wherein the valve is configured to be elastically restored and press the pressing members in a recessed direction of the recesses.

6. The medicinal liquid flow control apparatus of Claim 5, wherein the actuator is configured to be actuated in a state in which the recesses move in a direction transverse to the recessed direction thereof, and
wherein the plurality of pressing members are selectively accomodated in the plurality of recesses when the actuator is actuated.

7. The medicinal liquid flow control apparatus of Claim 6, wherein the valve is configured to open a corresponding branch flow path in a state in which the pressing members are accommodated in the recesses.

8. The medicinal liquid flow control apparatus of Claim 3, further comprising:
a guide member disposed between the valve and the plurality of pressing members to guide a movement of the plurality of pressing members.

9. The medicinal liquid flow control apparatus of Claim 1, further comprising:
a plurality of pressing members configured to correspond to the plurality of branch flow paths, respectively, and configured to be individually movable;
an actuator configured to move the pressing members; and
an operation member removably engaged with the actuator and configured to actuate the actuator in a state of being engaged with the actuator.

10. The medicinal liquid flow control apparatus of Claim 9, wherein the operation member is provided with an engaging portion, and the actuator is provided with a counterpart engaging portion in which the engaging portion is accommodated.

11. The medicinal liquid flow control apparatus of Claim 1, wherein the valve is configured to be elastically deformed by being pressed and elastically restored by being released.

12. The medicinal liquid flow control apparatus of Claim 1, further comprising:
a valve housing provided with a plurality of valve spaces covered by the valve, each of the valve spaces defining a portion of a corresponding one of the plurality of branch flow paths.

13. The medicinal liquid flow control apparatus of Claim 12, wherein the valve housing is provided with a plurality of valve holes that allow the plurality of valve spaces and the capillary flow paths of the plurality of transfer tubes to respectively communicate with each other.

14. The medicinal liquid flow control apparatus of Claim 13, further comprising:
a plurality of pressing members configured to correspond to the plurality of branch flow paths, respectively, and configured to be individually movable,
wherein the valve is configured to close the valve holes when pressed by the pressing members.

15. The medicinal liquid flow control apparatus of Claim 12, wherein one of the plurality of valve spaces is provided with a connection hole constituting a portion of the outlet flow path.

16. The medicinal liquid flow control apparatus of Claim 12, further comprising:
a communication flow path configured to communicate two valve spaces adjacent to each other among the plurality of valve spaces.

17. The medicinal liquid flow control apparatus of Claim 16, wherein one of the plurality of valve spaces is provided with a connection hole constituting a portion of the outlet flow path, and
wherein, in a state in which the valve opens the valve holes and in a state in which the valve closes the valve holes, the connection hole and the communication flow path are configured to maintain an opened state.

18. The medicinal liquid flow control apparatus of Claim 12, wherein the valve includes a plurality of protrusions that protrude toward the plurality of valve spaces and are configured to close the plurality of branch flow paths, respectively.

19. The medicinal liquid flow control apparatus of Claim 18, further comprising:
a plurality of pressing members configured to correspond to the plurality of branch flow paths, respectively, and configured to be individually movable,
wherein the valve is provided with a plurality of seating portions disposed on opposite sides to the plurality of protrusions so that the plurality of pressing members are seated on the seating portions, respectively.

20. The medicinal liquid flow control apparatus of Claim 1, further comprising:
an air pass filter disposed in the inlet flow path or the outlet flow path.
